(19) Europäisches Patentamt

European Patent Office

Office européen des brevets

(11) EP 0 781 129 B1

(12) EUROPÄISCHE PATENTSCHRIFT

(45) Veröffentlichungstag und Bekanntmachung des Hinweises auf die Patenterteilung:
02.07.2003 Patentblatt 2003/27

(21) Anmeldenummer: 95932741.2

(22) Anmeldetag: 14.09.1995

(51) Int Cl.⁷: **A61K 31/155**, A61K 9/20

(86) Internationale Anmeldenummer:
PCT/EP95/03610

(87) Internationale Veröffentlichungsnummer:
WO 96/008243 (21.03.1996 Gazette 1996/13)

(54) **PHARMAZEUTISCHE ZUBEREITUNG ENTHALTEND METFORMIN UND VERFAHREN ZU DEREN HERSTELLUNG**

PHARMACEUTICAL PREPARATION CONTAINING METFORMIN AND A PROCESS FOR PRODUCING IT

PREPARATION PHARMACEUTIQUE CONTENANT DE LA METFORMINE ET SON PROCEDE DE FABRICATION

(84) Benannte Vertragsstaaten:
**AT BE CH DE DK ES FR GB GR IE IT LI LU NL PT SE**
Benannte Erstreckungsstaaten:
**SI**

(30) Priorität: **14.09.1994 DE 4432757**

(43) Veröffentlichungstag der Anmeldung:
**02.07.1997 Patentblatt 1997/27**

(73) Patentinhaber: **Roche Diagnostics GmbH**
**68298 Mannheim (DE)**

(72) Erfinder:
• **MOECKEL, Jörn**
**D-69118 Heidelberg (DE)**
• **GABEL, Rolf-Dieter**
**D-68723 Schwetzingen (DE)**
• **WOOG, Heinrich**
**D-69514 Laudenbach (DE)**

(74) Vertreter: **Braun, Axel et al**
**F.Hoffmann-La Roche AG**
**Patent Department (PLP),**
**124 Grenzacherstrasse**
**4070 Basel (CH)**

(56) Entgegenhaltungen:
**EP-A- 0 283 369** **US-A- 3 621 097**

**Beschreibung**

Die Erfindung betrifft pharmazeutische Zubereitungen enthaltend Metformin-Hydrochlorid (im folgenden auch Metformin genannt) als Wirkstoff und einen Hydrokolloidbildner als Retardierungsmittel und ein Verfahren zu deren Herstellung.

Es ist bekannt, daß Metformin-Hydrochlorid ein Biguanidderivat ist (1,1-Dimethylbiguanid-Monohydrochlorid), das oral antidiabetisch wirkt. Metformin-Retardtabletten sind mit 850 mg Metformin-Hydrochlorid pro Filmtablette (Glucophage® retard) auf dem Markt. Da sich Metformin im Gegensatz zu anderen Wirkstoffen als Reinsubstanz nicht verpressen läßt (die Masse zerfällt nach dem Komprimieren unverändert), wurde bei diesen hochdosierten Retardtabletten auf gerüstbildende Hilfsstoffe wie Polyvinylacetat als Retardierungsmittel zurückgegriffen (Lipha, Fachinformation Glucophage®, August 1991; Bundesverband der Pharmazeutischen Industrie e.V., Hrsg., Rote Liste 1993, Edition Cantor, Aulendorf 1993). Die Wirkungsweise solcher Gerüsttabletten beruht darauf, daß das gut wasserlösliche Metformin im Magen-Darm-Trakt pH-unabhängig aus der Tablette herausdiffundiert, während das Tablettengerüst mit Überzug weitgehend unverändert wieder ausgeschieden wird.

Der Nachteil der Verwendung solcher gerüstbildenden Hilfsstoffe wie Polyvinylacetat beruht jedoch darauf, daß sie insbesondere beim Granulationsvorgang mit organischen Lösungsmitteln verarbeitet werden müssen, wobei das organische Lösungsmittel möglichst wieder vollständig entfernt werden muß, bevor das Granulat zu komprimierten pharmazeutischen Darreichungsformen weiterverarbeitet und beispielsweise zu Tabletten verpreßt wird.

Dokument EP-A-0 283 369 beschreibt die Verwendung von Metformin zur Behandlung von Erkrankungen des Mikro-Gefäßsystems.

Aufgabe der Erfindung war es, eine verbesserte pharmazeutische Zusammensetzung für den Wirkstoff Metformin zur Verfügung zu stellen. Insbesondere sollte die Darreichungsform den Wirkstoff Metformin mit einem möglichst hohen Wirkstoffanteil und einem Retardierungsmittel enthalten, wobei das Retardierungsmittel eine kontrollierte Freisetzung des Wirkstoffes bewirkt. Insbesondere sollte die neue pharmazeutische Zusammensetzung keine mit organischen Lösungsmittel zu verarbeitende Gerüstbildner enthalten, sondern auf Basis von wäßrig verarbeitbaren Substanzen aufgebaut sein.

Diese pharmazeutischen Zusammensetzungen sollten gut bzw. leicht komprimierbar sein, so daß sie sich zur Herstellung von festen pharmazeutischen Darreichungsformen, wie z.B. Tabletten, Dragees oder Komprimaten zur Abfüllung in Kapseln eignen. Bei der Herstellung von Tabletten oder anderen Komprimaten sollte das Gesamtgewicht maximal etwa 1200 - 1300 mg betragen, um die Therapiesicherheit (Patienten-Compliance) nicht zu gefährden, da größere orale Darreichungsformen vom Patienten häufig nicht in der vorgeschriebenen Regelmäßigkeit eingenommen werden.

Außerdem stellte sich die Aufgabe, bei der Verarbeitung des Granulats für diese hochdosierten Darreichungsformen, insbesondere bei der Herstellung von Tabletten, das im Fall von Metformin besonders ausgeprägte wirkstoffbedingte Problem des Deckelns zu lösen, um Ausbeuteverluste während der Produktion und Beeinträchtigungen der pharmazeutischen Qualität zu vermeiden. Als Deckeln wird das Ablösen von verpreßter Masse in Schichten vom hergestellten Preßling während des Verpressens oder kurz danach bezeichnet (Schepky G. in: Bruchhausen F. von et al.; Hrsg.; Hagers Handbuch der pharmazeutischen Praxis, Band 2, Methoden, 5. Aufl., Springer Verlag, Berlin 1991). Im Fall von Metfomin, insbesondere bei hochdosierten Wirkstoffgehalten im Granulat hat sich gezeigt, daß die Tendenz des Deckelns bei der Herstellung der Tabletten besonders hoch ist.

Die Ursachen für diese Tablettierprobleme können vielfältig und komplex sein. Deckeln kann ausgelöst werden durch ungenügende Bindemittelwirkung, zu geringe oder zu hohe Granulatfeuchtigkeit, ungeeignete Kristallformen, stark aerophile Stoffe, zu hohe Porosität, zu hohen Pulveranteil, zu starke interpartikulare Bindung zwischen den Granulatkörnern sowie durch ungeeignete Granulatformen. Als maschinenbedingte Faktoren können zu hohe Preßkraft, schlecht eingesetzte oder auch abgenutzte Werkzeuge, zu hohe Preßgeschwindigkeit und schlechte Entlüftung der Matrize (starrer Druck) zum Deckeln führen. Im Falle des Wirkstoffes Metformin hat sich jedoch gezeigt, daß die üblichen Möglichkeiten nicht ausreichen, um das Deckeln der Tablettiermasse befriedigend zu beherrschen. Bei der Herstellung der Tabletten konnte regelmäßig ein relativ hoher Anteil an fehlerhaften Tabletten festgestellt werden und die Tablettierung mußte aufgrund der hohen Ausschußraten abgebrochen werden.

Im vorliegenden Fall wird die Aufgabe der Erfindung gelöst, indem hochdosierte Metformin-haltige pharmazeutische Zusammensetzungen zur Verfügung gestellt werden, die als Retardierungsmittel einen Hydrokolloidbildner enthalten und die eine Restfeuchte in der pharmazeutischen Zusammensetzung von 0,5 - 3 Gew.-% aufweisen. Diese pharmazeutischen Zusammensetzungen können vorteilhaft unter Verwendung von wäßrigen Lösungsmitteln hergestellt werden, so daß organische Lösungsmittel nicht mehr benötigt werden. Außerdem sind diese Zusammensetzungen überraschenderweise gut komprimierbar. Sie eignen sich dadurch insbesondere zur Herstellung von festen pharmazeutischen Darreichungsformen, wie z.B. Tabletten, Dragees oder Kapseln, wobei diese mit Hilfe der üblichen Verarbeitungsmaschinen in technischem Maßstab und in guter Qualität sowie in hoher Ausbeute ohne größere Verluste infolge des unerwünschten Deckelns hergestellt werden können. Gegenstand der Erfindung ist demnach auch ein entsprechendes Verfahren zur Herstellung dieser festen Darreichungsformen, indem man die entsprechenden erfindungsgemäßen pharmazeutischen Zusammensetzungen in Form von Granulaten mit einer Restfeuchte von 0,5 - 3 Gew.-% einsetzt. Vorzugsweise beträgt die Restfeuche 1 - 2,5 Gew.-%, insbesondere 1,5 - 2 Gew.-%.

Überraschenderweise wurde ferner gefunden, daß im Falle des erfindungsgemäßen Granulats auf den sonst oft erforderlichen Zusatz von Feuchthaltemittel zur Einstellung einer konstanten Restfeuchte bis zum Komprimieren des Granulates verzichtet werden kann. Dies ist insbesondere deshalb vorteilhaft, da sich der Zusatz von Hilfsstoffen somit minimieren läßt und pharmazeutische Zusammensetzungen mit einem relativ hohen Wirkstoffgehalt erhalten werden. Außerdem haben diese Zusammensetzungen den Vorteil, daß sie für einen Zeitraum von zwei Tagen oder mehr (gerechnet von der Herstellung bis zur Verwendung des Granulates zur Tablettierung) bezüglich des Feuchtegehaltes lagerstabil sind, bevor sie komprimiert werden, ohne daß eine nachteilige Veränderung der Zusammensetzung feststellbar ist. Dies ist deshalb von Vorteil, da somit mehrere Teilansätze von Produktionschargen der pharmazeutischen Zusammensetzung hergestellt werden können, und diese dann zu einem späteren Zeitpunkt in einem gemeinsamen letzten Verfahrensschritt als preßfertige Masse abgemischt und zu festen pharmazeutischen Darreichungsformen verarbeitet werden können.

Weiterhin zeigte sich überraschenderweise, daß durch die Verwendung eines Hydrokolloidbildners insbesondere die für Metformin bekannte schlechte Komprimierbarkeit erstmals technisch in zufriedenstellender Weise beherrschbar war. Die erfindungsgemäße Lösung ermöglicht zudem, daß durch die Wahl des Hydrokolloidbildners als Retardierungsmittel und bei geeigneter Führung des Herstellprozesses (Einhalten der kritischen Restfeuchte von 0,5 - 3 % Gew.-%, insbesondere von 1 - 2,5 Gew.-% bzw. 1,5 - 2 Gew.-%) die gewünschte Retardierung und Komprimierbarkeit gewährleistet sind, obwohl der Anteil des Hydrokolloidbildners an der Rezepturzusammensetzung außergewöhnlich niedrig ist. Dies ist um so überraschender, als der überwiegende Anteil der Rezeptur (etwa 70 - 95 Gew.-%) durch den Wirkstoff gebildet wird, dessen Wassersorptionsvermögen sehr gering ist (bei einer relativen Feuchte von 90 % bindet der reine Wirkstoff lediglich 0,04 Gew.-% Wasser).

Der Gewichtsanteil des Wirkstoffes in der hochdosierten pharmazeutischen Zusammensetzung liegt im Bereich von mindestens 70 Gew.-%, vorzugsweise 80 - 95 Gew.-%, bezogen auf die pharmazeutische Zusammensetzung. Der Wirkstoff kann in Form von Säureadditionssalzen von anorganischen oder organischen Säuren, wie z.B. Salzsäure, Ameisensäure, Essigsäure, Äpfelsäure, Weinsäure oder Fumarsäure eingesetzt werden. Bevorzugt wird das Hydrochlorid-Salz eingesetzt.

Der Anteil der Hydrokolloidbildner an der pharmazeutischen Zusammensetzung beträgt bis zu 15 Gew.-%, vorzugsweise 4 - 10 Gew.-%, insbesondere etwa 6 - 8 Gew.-%.

Als Hydrokolloidbildner bzw. als hydrophile Quellstoffe im Sinne der Erfindung sind die üblichen hydrophilen Gelbildner geeignet, wie beispielsweise Cellulosederivate, Dextrine, Stärke, Polymere auf Kohlenhydratbasis, natürliche und hydrophile Gummen, Xanthane, Alginate, Gelatine, Polyacrylsäure, Polyvinylalkohol oder Polyvinylpyrrolidon. Im Falle der Cellulosederivate kommen bevorzugt die Alkyl- oder Hydroxyalkylcellulose-Derivate in Frage, wie z. B. Methylcellulose, Hydroxymethylcellulose, Hydroxyethylcellulose, Hydroxypropylcellulose, Methylhydroxyethylcellulose, Methylhydroxypropylcellulose oder Natriumcarboxymethylcellulose. In einer bevorzugten Ausführungsvariante der Erfindung kommt Methylhydroxypropylcellulose (MHPC) zum Einsatz. Die Hydrokolloidbildner können sowohl einzeln als auch in Gemischen von zwei oder mehreren Kolloidbildnern verwendet werden. Als geeignete polymere Kolloidbildner auf Cellulosebasis können die üblichen für pharmazeutische Zwecke geeigneten Polymere mit unterschiedlichem Substitutionsgrad und/oder unterschiedlichem Molekulargewicht, entsprechend einem unterschiedlichen Viskositätsgrad der wäßrigen Lösung, eingesetzt werden.

Die Verwendung von Hydrokolloidbildnern als Retardierungsmittel beruht auf der Eigenschaft der Hydrokolloidbildner, daß dieser bei Kontakt mit Freisetzungsmedium oder Verdauungssäften unter Quellung eine Gelmatrix ausbildet, die unter Erosion den Wirksoff freisetzt. Das Zusammenwirken von Hydrokolloidbildner-Menge und Viskositätsgrad bestimmt dabei den Freisetzungsverlauf. So kann beispielsweise mit einem hohen Anteil (70 - 95 %, beogen auf das Kerngeweicht einer Tablette) von Polyvinylalkohol niedriger oder mittlerer Viskositätsstufe Riboflavin über mehrere Stunden retardiert werden (Möckel J E., Lippold B. C., Pharm. Research, 1993, 10, 1066 - 1070).

Die unter Verwendung der erfindungsgemäßen pharmazeutischen Zusammensetzung hergestellten komprimierten Darreichungsformen, wie beispielsweise Metformin-Retardtablettenkerne können zusätzlich mit einer Filmhülle versehen werden. Die Filmhülle kann einerseits eine zusätzliche Retardierung bewirken, indem solche Filmmaterialien eingesetzt werden, die einen für diese Zwecke üblicherweise geeigneten Filmbildner darstellen. Andererseits kann die verwendete Filmhülle ein geschmacksneutralisierender Filmbildner sein, dem gegebenenfalls Farbstoffe zugesetzt werden können. Weiterhin ist beispielsweise auch der Einsatz von magensaftresistenten Filmen möglich. Der Gewichtsanteil der Filmhülle bezogen auf die fertige Tablette liegt im üblichen Bereich von 0,3 - 3,0 Gew.-%, vorzugsweise von 0,8 - 1,2 Gew.%. Als Filmbildner kommen übliche Filmbildner, wie beispielsweise Ethylcellulose, Poly-(methylmethacrylat)-Derivate (Eudragit®), aber auch lösliche Cellulosederivate wie Methylhydroxypropylcellulose und Cellulosederivate zur Ausbildung magensaftresistenter Filme, wie Celluloseacetatphthalat oder Methylhydroxypropylcellulosephthalat in Frage. Bevorzugt wird Ethylcellulose verwendet. Durch den gebildeten Film kann die Auflösung des Wirkstoffs verzögert werden. Als übliche Hilfsstoffe können in der Filmhülle Weichmacher, Porenbildner und Pigment enthalten sein.

Die erfindungsgemäße pharmazeutische Zusammensetzung kann auch zur Herstellung von komprimierten Kapselfüllmassen verwendet werden. Diese Komprimate bzw kompaktierten Granulate können dann in handelsübliche Kapseln mittels geeigneter Vorrichtungen gefüllt werden. Im Vergleich zu den sonst üblichen Metformin-haltigen Kapselfüllmassen haben diese kompaktierten Granulate bei gleichem Wirkstoffgehalt bzw. gleicher Dosierung den Vorteil, daß aufgrund ihres geringeren Volumens kleinere Kapseln verwendet werden können, die leichter von dem Patienten geschluckt werden können.

Die erfindungsgemäßen pharmazeutischen Darreichungsformen, wie z.B. Tabletten, enthalten - neben dem Wirkstoff, dessen Anteil an der Darreichungsform im Bereich von 70 - 95 Gew.-% liegen kann, (beispielsweise werden 850 mg der Wirkstoffes im Fall von Retardtabletten bevorzugt eingesetzt) und dem Retardierungsmittel - vorzugsweise 2 - 10 Gew.-% Bindemittel, bis zu 2 Gew.-%, vorzugsweise 0,1 - 0,3 Gew.-% Fließregulierungsmittel und bis zu 2 Gew.-%, vorzugsweise 0,4 - 1,1 Gew.-% Schmiermittel, jeweils bezogen auf das Gesamtgewicht der tablettierfertigen Masse bzw. des Tablettenkerns. Das Gewicht eines Tablettenkern liegt in der Regel zwischen 200 und 1300 mg, vorzugsweise im Bereich von weniger als 1200 mg, insbesondere von etwa 500 - 1000 mg. Als Fließregulierungsmittel kommen für die erfindungsgemäße Tablette übliche Mittel wie zum Beispiel kolloidales Siliciumdioxid in Frage. Als Schmiermittel sind beispielsweise Talkum oder Stearinsäure bzw. deren Alkali- oder Erdalkalisalze, insbesondere Magnesiumstearat, geeignet. Als Bindemittel können beispielsweise Cellulosederivate, speziell Alkyl- und Hydroxyalkyl-Cellulosen, insbesondere Methylcellulose, Hydroxymethylcellulose, Hydroxyethylcellulose, Hydroxypropylcellulose, Methylhydroxyethylcellulose, Methylhydroxypropylcellulose, Natriumcarboxymethylcellulose u.a., Dextrine, Stärken, speziell lösliche Stärken, andere Polymere auf Kohlenhydratbasis wie z.B. Galaktomannane, natürliche Gummen wie Gummi arabicum, Traganth, Sterculia, Acacia u.a., Xanthan, Alginate, Polyacrylsäure, Polyvinylalkohol und Polyvinylpyrrolidon eingesetzt werden. Bevorzugt wird Polyvinylpyrrolidon verwendet.

Die erfindungsgemäßen pharmazeutischen Darreichungsformen, wie z.B. Tabletten, werden hergestellt, indem man den Wirkstoff, das Retardierungsmittel oder einen Teil des Retardierungsmittels und gegebenenfalls weitere Hilfsstoffe trocken miteinander vermischt, mit Wasser oder einer wäßrigen Lösung eines Bindemittels feucht granuliert, die tablettierfertige Masse bis zu einer gewünschten Restfeuchte trocknet und danach gegebenenfalls den anderen Teil des Retardierungsmittels oder andere pharmazeutische Hilfsstoffe dem Granulat zumischt, so daß in dem letzten Verfahrensschritt eine Restfeuchte in der pharmazeutischen Zusammensetzung von 0,5 -3 Gew.-% erzielt wird. Die Bestimmung der Restfeuchte erfolgt nach bekannten analytischen Methoden der Aquametrie, beispielsweise durch die Bestimmung des Wassergehaltes mit Hilfe des Karl-Fischer-Reagenzes, oder anderen alternativen Bestimmungsverfahren. Bei der Feuchtgranulation kann auch ein Teil des Wirkstoffes, die verwendeten Hilfsstoffe sowie das Retardierungsmittel ganz oder teilweise in Wasser gelöst oder suspendiert vorliegen. Gegebenenfalls können auch mit Wasser mischbare organische Lösungsmittel, wie beispielsweise Aceton oder niedere Alkohole, wie Methanol oder Ethanol zugesetzt werden.

Die Einstellung der Restfeuchte erfolgt zweckmäßigerweise im Rahmen einer Trocknung im Wirbelschichtverfahren, wobei das feuchte Granulat so lange getrocknet wird, bis die gemessene Feuchtigkeit in der Abluft den zuvor im Rahmen einer Kalibrierung zur Restfeuchte im Trocknungsgut ermittelten Wert erreicht hat. Die so hergestellte Zusammensetzung wird anschließend in üblicher Weise zu pharmazeutischen Darreichungsformen verarbeitet und beispielsweise zu Tabletten verpreßt. Die Tabletten können mit üblichen Überzugsverfahren mit einem Film überzogen werden. Es wurde gefunden, daß die mit Hilfe des Hydrokolloidbildners eingestellte Restfeuchte von 0,5 - 3 Gew.-% gewährleistet, daß die tablettierfertige Masse über den gesamten, zur Herstellung großer Tabletten notwendigen Preßkraftbereich ohne Deckeln komprimierbar ist.

Der Wirkstoff kann ganz oder teilweise mit dem zur Retardierung eingesetzten Hydrokolloidbildner zu einem Granulat verarbeitet werden oder der Hydrokolloidbildner wird vollständig einem Hydrokolloidbildner-freien Granulat nach dessen Herstellung zugemischt. Eine zusätzlich bessere Tablettierbarkeit wird jedoch erreicht, wenn der Hydrokolloidbildner oder ein Teil davon mit dem Wirkstoff granuliert wird.

Das Überziehen der Tablette erfolgt nach üblichen Verfahren wie z. B. dem Dragierkessel- oder Wirbelbettverfahren.

Die erfindungsgemäßen retardierten Tabletten setzen Metformin über einen Zeitraum von 0,5 - 10 Stunden, vorzugsweise über 4 Stunden, kontrolliert frei (Abb. 1). Das Gewicht der Tabletten liegt dadurch, daß durch die Verwendung des Hydrokolloidbildners keine großen Mengen zusätzlicher Hilfsstoffe, insbesondere keine Feuchthaltemittel, wie z.B. Gycerol oder Sorbitol, notwendig sind, bei maximal 1200 mg, vorzugsweise unter 1000 mg.

Nachfolgend soll die Erfindung durch Ausführungsbeispiele verdeutlicht werden, ohne sie darauf einzuschränken.

Bei den folgenden Beispielen 1 - 6 wurde die Restfeuchte auf den erfindungsgemäßen Bereich eingestellt, bevor die pharmazeutische Zusammensetzung in Form einer preßfertigen Masse zu Tabletten verpreßt wurde. In den Beispielen 7 und 8 wurde die Restfeuchte auf einen Wert von weniger als 0,5 Gew.-% eingestellt. In diesen beiden Fällen mußte die Tablettierung aufgrund der hohen Verluste durch Deckeln abgebrochen werden.

**Beispiel 1:**

Hydrokolloidbildner: Methylhydroxypropylcellulose(MHPC). Der MHPC-Anteil kann variiert werden, z.B. von 40 - 95 mg.

Restfeuchte: 2.1 %

| Bestandteile | Tablette [mg] | preßfertige Masse [kg/l Mio St.] |
|---|---|---|
| Kern: | | |
| Metformin-Hydrochlorid | 850,00 | 850,00 |
| Methylhydroxypropylcellulose | 60,00 | 60,00 |
| Polyvidon | 38,00 | 38,00 |
| Magnesiumstearat | 5,00 | 5,00 |
| Kern gesamt: | 953,00 | 953,00 |
| Filmhülle: | | |
| Methylhydroxypropylcellulose | 20,00 | 20,00 |
| Ethylcellulose | 12,00 | 12,00 |
| Macrogol | 4,00 | 4,00 |
| Titandioxid | 4,00 | 4,00 |
| Hülle gesamt: | 40,00 | 40,00 |
| Filmtablette gesamt: | 993,00 | 993,00 |

Herstellung :

Die Herstellung des Granulats für eine Menge von etwa 1 Million Tabletten erfolgt in fünf Teilansätzen. Für jeden der fünf Teilansätze werden 170 kg Metformin-Hydrochlorid und 12 kg Methylhydroxypropylcellulose trocken miteinander gemischt und mit einer 10%igen wässrigen Bindemittellösung von Polyvidon in einem Mischer feucht granuliert. Danach wird das Granulat in einem Wirbelschichtgranulator getrocknet, bis es eine ausreichende Restfeuchte besitzt. Die funf Teilansätze werden vereinigt und mit 5 kg Magnesiumstearat gemischt. Die preßfertige Masse wird tablettiert. Die Tablettenkerne werden im Dragierkessel mit dem Film beschriebener Zusammensetzung überzogen.

Bei der aufgeführten Rezeptur wird die Restfeuchte auf 2,1 % eingestellt. Die Tablettierung verläuft entsprechend problemlos, d.h. eine Deckeln der hergestellten Tablettenmasse kann nicht festgestellt werden.

**Beispiel 2:**

Hydrokolloidbildner: Hydroxyethylcellulose

Restfeuchte: 2,0 %

| Bestandteile | Tablette [mg] | preßfertige Masse [kg/l Mio St.] |
|---|---|---|
| Kern: | | |
| Metformin-Hydrochlorid | 850,00 | 850,00 |
| Hydroxyethylcellulose | 70,00 | 70,00 |
| Polyvidon | 40,00 | 40,00 |
| Magnesiumstearat | 5,00 | 5,00 |
| Kern gesamt: | 965,00 | 965,00 |
| Filmhülle: | | |
| Methylhydroxypropylcellulose | 5,00 | 5,00 |
| Lactose | 5,00 | 5,00 |
| Ethylcellulose | 10,00 | 10, |

5

(fortgesetzt)

| Bestandteile | Tablette [mg] | preßfertige Masse [kg/l Mio St.] |
|---|---|---|
| Filmhülle: | | |
| Macrogol | 3,00 | 3,00 |
| Titandioxid | 3,00 | 3,00 |
| Hülle gesamt: | 26,00 | 26,00 |
| Filmtablette gesamt: | 991,00 | 991,00 |

Herstellung des Granulates und Weiterverarbeitung erfolgt analog zu Beispiel 1; die Tablettierung verläuft entsprechend problemlos.

**Beispiel 3:**

Hydrokolioidbildner: Natriumcarboxymethylcellulose
Restfeuchte: 2,1 %

| Bestandteile | Tablette [mg] | preßfertige Masse [kg/l Mio St] |
|---|---|---|
| Kern: | | |
| Metformin-Hydrochlorid | 850,00 | 850,00 |
| Natriumcarboxymethylcellulose | 80,00 | 80,00 |
| Polyvidon | 35,00 | 35,00 |
| Magnesiumstearat | 5,00 | 5,00 |
| Kern gesamt: | 970,00 | 970,00 |
| Filmhülle: | | |
| Methylhydroxypropylcellulose | 5,00 | 5,00 |
| Ethylcellulose | 10,00 | 10,00 |
| Macrogol | 4,00 | 4,00 |
| Titandioxid | 3,00 | 3,00 |
| Hülle gesamt: | 22,00 | 22,00 |
| Filmtablette gesamt: | 992,00 | 992,00 |

Herstellung des Granulats und Weiterverarbeitung erfolgt analog Beispiel 1; die Tablettierung verläuft entsprechend problemlos.

**Beispiel 4:**

Hydrokolloidbildner: Polyacrylsäure
Restfeuchte: 2,8 %

| Bestandteile | Tablette [mg] | preßfertige Masse [kg/l Mio St] |
|---|---|---|
| Kern: | | |
| Metformin-Hydrochlorid | 850,00 | 850,00 |
| Polyacrylsäure | 60,00 | 60,00 |
| Methylhydroxypopylcellulose | 30,00 | 30,00 |
| Magnesiumstearat | 5,00 | 5,00 |

(fortgesetzt)

| Bestandteile | Tablette [mg] | preßfertige Masse [kg/l Mio St] |
|---|---|---|
| Kern: | | |
| Kern gesamt: | 945,00 | 945,00 |
| Filmhülle: | | |
| Methylhydroxypropylcellulose | 10,00 | 10,00 |
| Ethylcellulose | 10,00 | 10,00 |
| Macrogol | 3,00 | 3,00 |
| Titandioxid | 3,00 | 3,00 |
| Hülle gesamt: | 26,00 | 26,00 |
| Filmtablette gesamt: | 971,00 | 971,00 |

Herstellung und Weiterverarbeitung des Granulats erfolgt analog zu Beispiel 1. Abweichend dient hier Methylhydroxypropylcellulose als Bindemittel. Die Tablettierung erfolgt problemlos.

**Beispiel 5:**

Hydrokolloidbildner: Hydroxypropylcellulose
Restfeuchte: 1.95 %

| Bestandteile | Tablette [mg] | preßfertige Masse [kg/l Mio St] |
|---|---|---|
| Kern: | | |
| Metformin-Hydrochlorid | 850,00 | 850,00 |
| Hydroxypropylcellulose | 60,00 | 60,00 |
| Polyvidon | 40,00 | 40,00 |
| Magnesiumstearat | 5,00 | 5,00 |
| Kern gesamt: | 955,00 | 955,00 |
| Filmhülle: | | |
| Poly(ethylacrylat-methylmethacrylat)-Dispersion 30% | 6,00* | 6,00* |
| Talk | 1,20 | 1,20 |
| Antischaummittel | 0,07 | 0,07 |
| Hülle gesamt: | 7,27 | 7,27 |
| Filmtablette gesamt: | 962,270 | 962,270 |

*Mengenangabe bezogen auf die Trockensubstanz

Herstellung und Weiterverarbeitung des Granulats erfolgt analog zu Beispiel 1. Abweichend wird hier der Hydrokolloidbildner Hydroxypropylcellulose nicht mitgranuliert sondern dem fertigen Granulat trocken zugemischt.

**Beispiel 6:**

Hydrokolloidbildner: Methylhydroxypropylcellulose
Restfeuchte: 2,0 %
Im folgenden Beispiel wird auf die Verwendung eines zusätzlichen Bindemittels ganz verzichtet, die eingesetzte Methylhydroxypropylcellulose übernimmt gleichzeitig die Funktion von Binde- und Retardierungsmittel.

| Bestandteile | Tablette [mg] | preßfertige Masse [kg/l Mio St.] |
|---|---|---|
| Kern: | | |
| Metformin-Hydrochlorid | 850,00 | 850,00 |
| Methylhydroxypropylcellulose | 100,00 | 100;00 |
| Magnesiumstearat | 5.00 | 5,00 |
| Kern gesamt: | 955,00 | 955,00 |
| Filmhülle: | | |
| Methylhydroxypropylcellulose | 20,00 | 20,00 |
| Ethylcellulose | 12,00 | 12,00 |
| Macrogol | 4,00 | 4,00 |
| Titandioxid | 4,00 | 4,00 |
| Hülle gesamt: | 40,00 | 40,00 |
| Filmtablette gesamt: | 995,00 | 995,00 |

Herstellung:

Die Herstellung des Granulats erfolgt in 5 Teilansätzen. Für jeden der fünf Teilansätze werden 170 kg des Wirkstoffs Metformin-Hydrochlorid mit 18 kg Methylhydroxypropylcellulose im Wirbelschichtgranulierer vorgelegt. 2 kg Mehylhydroxypropylcellulose werden in 50 l Wasser gelöst. Die trockene Mischung wird mit der Bindemittel lösung im Wirbelschichtgranulierer granuliert und anschließend getrocknet. Die fünf Teilansätze werden vereinigt und mit 5 kg Magnesiumstearat gemischt. Diese preßfertige Masse wird tablettiert. Auf die Tablettenkerne wird im Dragierkessel der Film beschriebener Zusammensetzung aufgetragen.

**Beispiel 7**

Hydrokolloidbildner: Methylhydroxypropylcellulose
Restfeuchte: 0,49 %
Bei der untenstehenden Rezeptur wurde eine Feuchte von 0,49 % erhalten. Aufgrund des zu hohen Verlustes durch Deckeln mußte die Tablettierung abgebrochen werden.

| Bestandteile | Tablette [mg] |
|---|---|
| Kern: | |
| Metformin-Hydrochlorid | 850,00 |
| Methylhydroxypropylcellulose | 40,00 |
| Polyvidon | 38,00 |
| Magnesiumstearat | 5,00 |
| Kern gesamt: | 953,00 |
| Filmhülle: | |
| Methylhydroxypropylcellulose | 20,00 |
| Ethylcellulose | 12,00 |
| Macrogol | 4,00 |
| Titandioxid | 4,00 |
| Hülle gesamt: | 40,00 |
| Filmtablette gesamt: | 993,00 |

**Beispiel 8**

Hydrokolloidbildner: Gelatine
Restfeuchte: 0,48 %

Bei der untenstehenden Rezeptur wurde eine Feuchte von 0,48 % erhalten. Aufgrund des zu hohen Verlustes durch Deckeln mußte die Tablettierung abgebrochen werden.

| Bestandteile | [mg] |
|---|---|
| Kern: | |
| Metformin-Hydrochlorid | 850,00 |
| Lactose | 70,00 |
| Gelatine | 40,00 |
| Siliciumdioxid, hochdispers | 2,00 |
| Magnesiumstearat | 2,50 |
| Kern gesamt: | 964,50 |
| Filmhülle: | |
| Methylhydroxypropylcellulose | 10,00 |
| Ethylcellulose | 9,00 |
| Diethylphthalat | 3,00 |
| Titandioxid | 3,00 |
| Hülle gesamt: | 25,00 |
| Filmtablette gesamt | 989,5 |

**Patentansprüche**

1.  Pharmazeutische Zusammensetzung enthaltend Metformin als Wirkstoff und einen Hydrokolloidbildner als Retardierungsmittel sowie gegebenenfalls pharmazeutisch übliche Hilfsstoffe, wobei die Restfeuchte in der pharmazeutischen Zusammensetzung 0,5 - 3 Gew.-% beträgt.

2.  Pharmazeutische Zusammensetzung nach Anspruch 1, **dadurch gekennzeichnet, daß** der Wirkstoffgehalt von Metformin mindestens 70 % beträgt.

3.  Pharmazeutische Zusammensetzung nach Anspruch 1 oder 2, **dadurch gekennzeichnet, daß** die Menge des Hydrokolloidbildners 4 - 15 Gew.-% beträgt.

4.  Pharmazeutische Zusammensetzung nach einem der Ansprüche 1 - 3, **dadurch gekennzeichnet, daß** der Hydrokolloidbildner ausgewählt ist aus Cellulosederivaten, Dextrinen, Stärken, Polymeren auf Kohlenhydratbasis, natürliche Gummen, Xanthan, Alginate, Gelatine, Polyacrylsäure, Polyvinylalkohol und Polyvinylpyrrolidon.

5.  Pharmazeutische Zusammensetzung nach Anspruch 4, **dadurch gekennzeichnet, daß** der Hydrokolloidbildner ein Cellulosederivat ist, insbesondere eine Alkyl- oder Hydroxyalkylcellulose.

6.  Pharmazeutische Zusammensetzung nach Anspruch 5, **dadurch gekennzeichnet, daß** der Hydrokolloidbildner ausgewählt ist aus Methylcellulose, Hydroxymethylcellulose, Hydroxyethylcellulose, Hydroxypropylcellulose, Methylhydroxyethylcellulose, Methylhydroxypropylcellulose oder Natriumcarboxymethylcellulose.

7.  Pharmazeutische Zusammensetzung nach einem der Ansprüche 1 - 6 enthaltend 3 - 5 Gew.% Bindemittel, bis zu 2 Gew.% Fließregulierungsmittel und bis zu 2 Gew.% Schmiermittel.

8.  Pharmazeutische Zusammensetzung nach einem der Ansprüche 1 - 6 zur Herstellung von komprimierten festen pharmazeutischen Darreichungsformen, insbesondere Tabletten oder Komprimaten zur Abfüllung in Kapseln.

9.  Pharmazeutische Darreichungsform in Form von Tabletten oder Komprimaten zur Abfüllung in Kapseln enthaltend Metformin als Wirkstoff und einen Hydrokolloidbildner als Retardierungsmittel mit einer Restfeuchte von 0,5 - 3 Gew.-% bezogen auf das Gewicht des Tablettenkerns oder der Kapselfüllmasse.

10. Pharmazeutische Darreichungsform nach Anspruch 9 in Form einer Tablette mit einem Endgewicht unter 1300 mg.

11. Verfahren zur Herstellung von pharmazeutischen Zusammensetzungen enthaltend Metformin als Wirkstoff und einen Hydrokolloidbildner als Retardierungsmittel sowie gegebenenfalls weitere pharmazeutisch übliche Hilfsstoffe, **dadurch gekennzeichnet, daß** man den Wirkstoff und das Retardierungsmittel oder ein Teil davon mit einem wäßrigen, gegebenenfalls bindemittelhaltigen Lösungsmittel granuliert, gegebenenfalls den anderen Teil des Retardierungsmittels oder andere pharmazeutisch übliche Hilfsmittel dem Granulat zumischt, und anschließend das Granulat bis zu einer Restfeuchte von 0,5 - 3 Gew.% trocknet.

12. Verfahren nach Anspruch 11, **dadurch gekennzeichnet, daß** das Granulat zu Tabletten verpreßt wird, die anschließend gegebenenfalls mit einer Filmhülle überzogen werden.

13. Verfahren nach Anspruch 11, **dadurch gekennzeichnet, daß** das Granulat kompaktiert wird und in Kapseln abgefüllt wird.

14. Verfahren nach Anspruch 11, **dadurch gekennzeichnet, daß** als Hydrokolloidbildner Methylhydroxypropylcellulose eingesetzt wird.

15. Verfahren nach Anspruch 11, **dadurch gekennzeichnet, daß** man zur Herstellung des Granulats bis zu 2 Gew.-% Fließregulierungsmittel, bis zu 2 Gew.-% Schmiermittel und bis zu 5 Gew.-% Bindemittel einsetzt, bezogen auf die fertige pharmazeutische Zusammensetzung.

16. Verwendung von pharmazeutischen Zusammensetzungen nach einem der Ansprüche 1 - 8 zur Herstellung von komprimierten pharmazeutischen Darreichungsformen, insbesondere von Tabletten oder Komprimaten zur Abfüllung in Kapseln.

17. Verfahren zur Herstellung eines leicht komprimierbaren pharmazeutischen Granulates enthaltend Metformin als Wirkstoff und einen Hydrokolloidbildner als Retardierungsmittel, **dadurch gekennzeichnet, daß** das Granulat vor der Komprimierung auf eine Restfeuchte von 0,5 - 3 Gew.-% getrocknet wird.

**Claims**

1. A pharmaceutical composition containing metformin as the active substance and a hydrocolloid-former as a retardant as well as, if desired, conventional pharmaceutical adjuvants, with the residual moisture content in the pharmaceutical composition being 0.5-3 wt.%.

2. A pharmaceutical composition according to claim 1, **characterized in that** the metformin active substance content is at least 70%.

3. A pharmaceutical composition according to claim 1 or 2, **characterized in that** the amount of hydrocolloid-former is 4-15 wt.%.

4. A pharmaceutical composition according to any one of claims 1-3, **characterized in that** the hydrocolloid-former is selected from cellulose derivatives, dextrins, starches, carbohydrate-based polymers, natural gums, xanthan, alginates, gelatin, polyacrylic acid, polyvinyl alcohol and polyvinylpyrrolidone.

5. A pharmaceutical composition according to claim 4, **characterized in that** the hydrocolloid-former is a cellulose derivative, especially an alkyl or hydroxyalkyl cellulose.

6. A pharmaceutical composition according to claim 5, **characterized in that** the hydrocolloid-former is selected from methyl cellulose, hydroxymethyl cellulose, hydroxyethyl cellulose, hydroxypropyl cellulose, methylhydroxyethyl cellulose, methylhydroxypropyl cellulose or sodium carboxymethyl cellulose.

7. A pharmaceutical composition according to any one of claims 1-6 containing 3-5 wt.% binder, up to 2 wt.% flow regulating agent and up to 2 wt.% lubricant.

8. A pharmaceutical composition according to any one of claims 1-6, for the production of compressed solid pharmaceutical administration forms, especially tablets or comprimates for filling into capsules.

9. A pharmaceutical administration form in the form of tablets or comprimates for filling into capsules containing metformin as the active substance and a hydrocolloid-former as a retardant having a residual moisture content of 0.5-3 wt.% based on the weight of the tablet core or the capsule fill mass.

10. A pharmaceutical administration form according to claim 9 in the form of a tablet with a final weight below 1300 mg.

11. A process for the production of pharmaceutical compositions containing metformin as the active substance and a hydrocolloid-former as a retardant as well as, if desired, additional conventional pharmaceutical adjuvants, **characterized by** granulating the active substance and the retardant or a part thereof with an aqueous solvent which optionally contains a binder, if appropriate admixing the other part of the retardant or other conventional pharmaceutical adjuvants with the granulate and subsequently drying the granulate to a residual moisture content of 0.5-3 wt.%.

12. A process according to claim 11, **characterized in that** the granulate is pressed to tablets which, if desired, are subsequently coated with a film envelope.

13. A process according to claim 11, **characterized in that** the granulate is compacted and filled into capsules.

14. A process according to claim 11, **characterized in that** methylhydroxypropyl cellulose is used as the hydrocolloid-former.

15. A process according to claim 11, **characterized in that** for the production of the granulate up to 2 wt.% flow regulating agent, up to 2 wt.% lubricant and up to 5 wt.% binder are used based on the finished pharmaceutical composition.

16. The use of pharmaceutical compositions according to any one of claims 1-8 for the production of compressed pharmaceutical administration forms, especially of tablets or comprimates for filling into capsules.

17. A process for the production of a readily compressible pharmaceutical granulate containing metformin as the active substance and a hydrocolloid-former as a retardant, **characterized by** drying the granulate to a residual moisture content of 0.5-3 wt.% before the compression.

**Revendications**

1. Composition pharmaceutique contenant de la metformine comme agent actif et un formateur d'hydrocolloïde comme agent retardateur ainsi qu'éventuellement des agents auxiliaires usuels sur le plan pharmaceutique, l'humidité résiduelle dans le composé pharmaceutique étant de 0,5 à 3 % en poids.

2. Composition pharmaceutique selon la revendication 1, **caractérisé en ce que** la teneur en agent actif de la metformine est d'au moins 70 %.

3. Composition pharmaceutique selon la revendication 1 ou 2, **caractérisé en ce que** la quantité de formateur d'hydrocolloïde est de 4 à 15 % en poids.

4. Composition pharmaceutique selon l'une quelconque des revendications 1 à 3, **caractérisé en ce que** le formateur d'hydrocolloïde est choisi à partir de dérivés de cellulose, de dextrines, d'amidons, de polymères à base de carbohydrates, de caoutchoucs naturels, de xanthane, d'alginates, de gélatine, d'acide polyacrylique, d'alcool polyvinylique et de pyrrolidon de polyvinyle.

5. Composition pharmaceutique selon la revendication 4, **caractérisé en ce que** le formateur d'hydrocolloïde est un dérivé de cellulose, en particulier une cellulose d'alkyle ou une cellulose d'hydroxyalkyle.

**6.** Composition pharmaceutique selon la revendication 5, **caractérisé en ce que** le formateur d'hydrocolloïde est choisi à partir de cellulose méthylique, de cellulose hydroxyméthylique, de cellulose hydroxyéthilique, de cellulose hydroxypropylique, de cellulose méthylhydroxyéthylique, de cellulose méthylhydroxypropylique et de cellulose carboxyméthylique de sodium.

**7.** Composition pharmaceutique selon l'une quelconque des revendications 1 à 6 contenant 3 à 5 % en poids de liant, jusqu'à 2 % en poids d'agent de régulation d'écoulement et jusqu'à 2 % en poids de lubrifiant.

**8.** Composition pharmaceutique selon l'une quelconque des revendications 1 à 6 pour la fabrication de présentation pharmaceutiques solides et comprimées, en particulier des comprimés ou des produits comprimés à mettre dans des capsules.

**9.** Présentation pharmaceutiques sous la forme de comprimés ou de produits comprimés à mettre dans des capsules, contenant de la metformine comme agent actif et un formateur d'hydrocolloïde comme agent de retardement avec une humidité résiduelle de 0,5 à 3 % en poids par rapport au poids du noyau du comprimé ou de la masse de remplissage de la capsule.

**10.** Présentation pharmaceutique selon la revendication 9 sous la forme d'un comprimé avec un poids final inférieur à 1300 mg.

**11.** Procédé pour la fabrication de compositions pharmaceutiques contenant de la metformine comme agent actif et un formateur d'hydrocolloïde comme agent de retardement ainsi qu'éventuellement d'autres agents auxiliaires usuels sur le plan pharmaceutique, **caractérisé en ce qu'**on granule l'agent actif et l'agent de retardement ou une partie de celui-ci avec un solvant aqueux et éventuellement contenant du liant, on ajoute au granulat éventuellement l'autre partie de l'agent de retardement ou d'autres agents auxiliaires usuels sur le pharmaceutique et on fait sécher ensuite le granulat jusqu'à une humidité résiduelle de 0,5 à 3 % en poids.

**12.** Procédé selon la revendication 11, **caractérisé en ce que** le granulat est compacté en comprimés, qui sont recouverts ensuite éventuellement d'une enveloppe de film.

**13.** Procédé selon la revendication 11, **caractérisé en ce que** le granulat est compacté et mis dans des capsules.

**14.** Procédé selon la revendication 11, **caractérisé en ce qu'**on utilise de la cellulose méthylhydroxypropylique comme formateur d'hydrocolloïde.

**15.** Procédé selon la revendication 11, **caractérisé en ce qu'**on utilise pour la fabrication du granulat jusqu'à 2 % en poids d'agent de régulation d'écoulement, jusqu'à 2 % en poids de lubrifiant et jusqu'à 5 % en poids de liant, par rapport à la composition pharmaceutique finie.

**16.** Utilisation de compositions pharmaceutiques selon l'une quelconque des revendications 1 à 8 pour la fabrication de présentations pharmaceutiques comprimées, en particulier de comprimés ou de produits comprimés à mettre dans des capsules.

**17.** Procédé pour la fabrication d'un granulat pharmaceutique pouvant être facilement comprimé contenant de la metformine comme agent actif et un formateur d'hydrocolloïde comme agent de retardement, **caractérisé en ce que** le granulat est séché avant la compression jusqu'à une humidité résiduelle de 0,5 à 3 % en poids.